# EUROPÄISCHE PATENTSCHRIFT

(11) **EP 4 217 734 B1**
(45) Veröffentlichungstag und Bekanntmachung des Hinweises auf die Patenterteilung: **08.10.2025**
(21) Anmeldenummer: 21755945.9
(22) Anmeldetag: 06.08.2021
(51) Int. Cl.: G01N 33/00, G01N 27/18, H01M 8/0432, H01M 8/0438, H01M 8/0444

(54) **SENSOR ZUR ERFASSUNG MINDESTENS EINER EIGENSCHAFT EINES FLUIDEN MEDIUMS IN MINDESTENS EINEM MESSRAUM**
SENSOR FOR DETECTING AT LEAST ONE PROPERTY OF A FLUID MEDIUM IN AT LEAST ONE MEASURING CHAMBER
CAPTEUR POUR DÉTECTER AU MOINS UNE PROPRIÉTÉ D'UN MILIEU FLUIDE DANS AU MOINS UNE CHAMBRE DE MESURE

(30) Priorität: 23.09.2020 DE 102020211893
(43) Veröffentlichungstag der Anmeldung: 02.08.2023
(73) Patentinhaber: Robert Bosch GmbH, 70442 Stuttgart (DE)
(72) Erfinder: FUCHS, Tino, 72076 Tuebingen (DE)
(86) Internationale Anmeldenummer: PCT/EP2021/072035
(87) Internationale Veröffentlichungsnummer: WO 2022/063476

(56) Entgegenhaltungen:
- EP-A1- 3 540 420
- EP-A2- 1 621 882
- WO-A1-00/54237
- US-A1- 2013 311 108
- BOON-BRETT L ET AL: "Identifying performance gaps in hydrogen safety sensor technology for automotive and stationary applications", INTERNATIONAL JOURNAL OF HYDROGEN ENERGY, ELSEVIER, AMSTERDAM, NL, vol. 35, no. 1, 13 November 2009 (2009-11-13), pages 373 - 384, XP026816701, ISSN: 0360-3199, [retrieved on 20091113]
- HBERT T ET AL: "Hydrogen sensors A review", SENSORS AND ACTUATORS B: CHEMICAL, ELSEVIER BV, NL, vol. 157, no. 2, 21 April 2011 (2011-04-21), pages 329 - 352, XP028233496, ISSN: 0925-4005, [retrieved on 20110429], DOI: 10.1016/J.SNB.2011.04.070

## Beschreibung

### Stand der Technik

Aus dem Stand der Technik ist eine Vielzahl von Sensoren, Sensorelementen und Verfahren zur Erfassung mindestens einer Eigenschaft eines fluiden Mediums in einem Messraum bekannt. Dabei kann es sich grundsätzlich um beliebige Eigenschaften eines gasförmigen oder flüssigen fluiden Mediums handeln, wobei eine oder mehrere Eigenschaften erfasst werden können. Die Erfindung wird im Folgenden, ohne Beschränkung weiterer Ausführungsformen und Anwendungen, insbesondere unter Bezugnahme auf Sensorelemente zur Erfassung eines Gases, insbesondere eines H₂-Anteils in einem Messgas, beschrieben.

Sensorelemente der hier beschriebenen Art finden Anwendung in einer Vielzahl von Gebieten, beispielsweise in der Automobiltechnik, der Verfahrenstechnik, der Chemie und dem Maschinenbau, insbesondere zur Bestimmung von Gaskonzentrationen. So spielt beispielsweise die Bestimmung von Wasserstoffkonzentrationen, beispielsweise in einem Luft-Wasserstoff-Gemisch, bei der Anwendung von Wasserstoff-Brennstoffzellen-Systemen eine große Rolle. Hierbei sind auch sicherheitsrelevante Anwendungen zu nennen. Ein Luft-Wasserstoff-Gemisch wird etwa bei einem Wasserstoffanteil von 4 % zündungsfähig. Sensorelemente zur Erfassung von Wasserstoff können beispielsweise in Wasserstoff-Brennstoffzellenfahrzeugen zum Einsatz kommen, um beispielsweise aufgrund von Beschädigung oder Defekt austretenden Wasserstoff zu detektieren und, durch eine Kopplung an entsprechende Systeme, Warnsignale und/oder Schutzmaßnahmen auszulösen. Daher werden pro Brennstoffzellenfahrzeug mehrere Wasserstoffsensoren benötigt, die entweder im Abgasstrang angebracht werden (exhaust) oder unter atmosphärischen Bedingungen arbeiten (ambient).

Für derartige Wasserstoffsensoren kann man auf eine Vielzahl von Messprinzipien zurückgreifen. Dazu gehören u.a. folgende Messprinzipien: Wärmeleitung, katalytischer Pellistor, elektrochemische Zelle, halbleitendes Metalloxid, Chemiresistor, Feldeffekt Transistor.

Trotz der Vorteile der aus dem Stand der Technik bekannten Sensorelemente zur Erfassung mindestens einer Eigenschaft eines fluiden Mediums beinhalten diese noch Verbesserungspotenzial. Für die Verwendung in Automobiltechnik muss ein solcher Wasserstoffsensor bestimmte Anforderungen erfüllen. Die zuvor genannten Messprinzipien besitzen bezüglich dieser Anforderungen jeweils bestimmte Mängel bzw. Nachteile. So weisen derartige Sensorelemente überwiegend eine unzureichende Reaktionszeit, einen Messbereich oberhalb des minimalen Messbereichs und/oder eine Querempfindlichkeit gegenüber weiteren Komponenten wie Helium oder flüchtigen organischen Komponenten auf. Außerdem basieren diese teilweise auf einer teuren Aufbau- und Verbindungstechnik.

Aus der US 2013/311108 A1 ist ein Sensor zur Erfassung mindestens einer Eigenschaft eines fluiden Mediums in mindestens einem Messraum bekannt, der ein erstes Sensorelement zum Erfassen einer Wärmeleitfähigkeit des fluiden Mediums und zum Ausgeben eines ersten Messsignals, ein zweites Sensorelement, das ein halbleitendes Metalloxid aufweist und das zum Ausgeben eines zweiten Messsignals ausgebildet ist, und ein drittes Sensorelement zum Erfassen einer physikalischen Eigenschaft des fluiden Mediums aufweist, wobei sich das dritte Sensorelement hinsichtlich der erfassten physikalischen Eigenschaft von dem ersten Sensorelement und dem zweiten Sensorelement unterscheidet und zum Ausgeben eines dritten Messsignals ausgebildet ist. Ferner sind elektronische Mittel zum Auswerten des ersten Messsignals, des zweiten Messsignals und dritten Messsignals und zum Ausgeben von Steuersignalen zum Verändern von Betriebsparametern des ersten Sensorelements, des zweiten Sensorelements und/oder des dritten Sensorelements mittels eines Algorithmus vorgesehen sind.

Der technische Artikel L. Boon-Brett et Al: "Identifying performance gaps in hydrogen safety sensor technology for automotive and stationary applications", erschienen im International Journal of Hydrogen Energy 35 (2010), Seiten 373-384, offenbart ein Multi-Typ Sensorsystem aus MOx-Sensoren und Sensoren basierend auf der Änderung der thermischen Leitfähigkeit, sowie einem dritten Sensor in Form eines Drucksensors.

Die EP 1 621 882 A2 offenbart eine Kombination eines Metalloxidsensors, eines Wärmeleitsensors und eines Temperatursensors.

Die EP 3 540 420 A1 offenbart eine Sensoreinheit bestehend aus einem TCD, einem Metalloxidsensor und einem Temperatursensor.

### Offenbarung der Erfindung

Im Rahmen der vorliegenden Erfindung wird ein Sensor zur Erfassung mindestens einer Eigenschaft eines fluiden Mediums in einem Messraum vorgeschlagen, welcher die Nachteile bekannter Sensoren zur Erfassung mindestens einer Eigenschaft eines fluiden Mediums in einem Messraum zumindest weitgehend vermeidet, und der ausreichend Empfindlichkeit, Messbereich, Reaktionszeit und Selektivität bezüglich der Anforderungen in der Automobiltechnik bietet.

Ein erfindungsgemäßer Sensor zur Erfassung mindestens einer Eigenschaft eines fluiden Mediums in mindestens einem Messraum, insbesondere zur Erfassung eines H₂-Anteils in einem Messgas, umfasst mindestens:
ein erstes Sensorelement, das zum Erfassen einer Wärmeleitfähigkeit des fluiden Mediums und zum Ausgeben eines ersten Messsignals ausgebildet ist,
ein zweites Sensorelement, das ein halbleitendes Metalloxid aufweist und das zum Ausgeben eines zweiten Messsignals ausgebildet ist,
ein drittes Sensorelement zum Erfassen einer physikalischen Eigenschaft des fluiden Mediums, wobei sich das dritte Sensorelement hinsichtlich der erfassten physikalischen Eigenschaft von dem ersten Sensorelement und dem zweiten Sensorelement unterscheidet und zum Ausgeben eines dritten Messsignals ausgebildet ist, und
eine elektronische Auswerteeinheit zum Auswerten des ersten Messsignals, des zweiten Messsignals und dritten Messsignals, wobei die elektronische Auswerteeinheit weiterhin zum Ausgeben von Steuersignalen an das erste Sensorelement, das zweite Sensorelement und das dritte Sensorelement und zum Verändern von Betriebsparametern des ersten Sensorelements, des zweiten Sensorelements und/oder des dritten Sensorelements mittels eines Algorithmus ausgebildet ist. Erfindungsgemäß umfasst der Algorithmus ein künstliches neuronales Netzwerk, wobei die elektronische Auswerteeinheit zum Ausgeben von Korrektursignalen an das erste Sensorelement, das zweite Sensorelement und das dritte Sensorelement zum Verändern der Betriebsparameter ausgebildet ist.

Die Information über die zu messende Eigenschaft des fluiden Mediums, wie beispielsweise eine H₂-Konzentration im zugeführten Messgas wird hauptsächlich durch das erste Sensorelement in Form eines Wärmeleitfähigkeit-Sensorelement und das zweite Sensorelement in Form eines MOX-Sensorelement (MOX = Metalloxid) generiert. Die Wärmeleitfähigkeit eines Gases ist invers proportional zur Wurzel der Masse der Gasmoleküle, so dass Gase mit leichten Atomen wie z.B. H₂-Moleküle oder He-Atome deutlich höhere Wärmeleitfähigkeit haben als Luft, die im Wesentlichen aus N₂ und O₂ Molekülen besteht. Je höher die gemessene Wärmeleitfähigkeit, desto größer der Anteil an leichten Molekülen. Das MOX-Sensorelement besteht aus einem halbleitenden Metall-Oxid wie z.B. SnO₂ oder WO₃, dessen elektrischer Widerstand sich verringert, wenn ein chemisch reduzierend wirkendes Gas wie z.B. Wasserstoff, Methan oder Wasserdampf in Luft enthalten ist. Über den gemessenen elektrischen Widerstand, lässt sich dadurch der Gehalt an reduzierenden Gasen in Luft bestimmen. Allerdings sind die Signale von Wärmeleitfähigkeit- und MOX-Sensorelement nicht eindeutig durch die Wasserstoffkonzentration in Luft bestimmt, denn auch andere Gase in Luft können zum gleichen Messergebnis führen. Man spricht hier von Querempfindlichkeit bezüglich anderer Gase, welche die absolute Sensor-Genauigkeit begrenzt. Um eine angestrebte Genauigkeit von 0.1 %vol. für H₂ bei allen nur denkbaren Luftzusammensetzungen gewährleisten zu können, sind Korrekturen in der Signalverarbeitung notwendig, indem zusätzliche Messgrößen erfasst und ausgewertet werden. Erfindungsgemäß wird daher mindestens ein weiteres Sensorelement zur Erfassung einer weiteren physikalischen Eigenschaft des fluiden Mediums vorgeschlagen. Beispielsweise werden weitere Sensorelemente für die relative Luftfeuchtigkeit, die Gastemperatur und den Gasdruck im H₂-Sensor integriert, deren Signale in der elektronischen Auswerteeinheit als zentrale elektronische Signalverarbeitungseinheit berücksichtigt werden. Diese Berücksichtigung erfolgt allerdings nicht in Form eines Kennlinienfeldes, sondern durch Methoden des Machine Learnings wie z.B. durch Trainieren eines Neuronalen Netzes.

Die elektronische Auswerteeinheit ist erfindungsgemäß zum Verändern von Betriebsparametern des ersten Sensorelements, des zweiten Sensorelements und/oder des dritten Sensorelements mittels eines Algorithmus ausgebildet , wobei der Algorithmus ein künstlich neuronales Netzwerk umfasst.

Das Training des neuronalen Netzes erfolgt vor Auslieferung des Sensors. Dabei wird erfindungsgemäß von den Methoden des Transfer-Learnings gebraucht gemacht, in der Art, dass das komplette zeitaufwändige Training des neuronalen Netzes nur an wenigen Produktsensoren erfolgt. Die übrigen Sensor-Produkte erhalten diese Knoten-Parameter bei ihrer Programmierung, und durchlaufen nur ein kurzes Training zum Zweck eines individuellen Fine-Tunings. Das Training besteht darin, den Sensor nacheinander Luft mit verschiedenen Anteilen an Gasen wie vor allem H₂ oder Störgasen wie z.B. He oder CH₄ bei verschiedenen relativen Feuchtigkeiten und unterschiedlichen Luftdrücken und Temperaturen auszusetzen, und die eingestellten Parameter für die Gaskonzentrationen, relative Luftfeuchte, Luftdruck und Temperatur als Trainingsdaten für das neuronale Netz zu verwenden.

Das dritte Sensorelement kann ausgebildet sein zur Erfassung mindestens einer physikalischen Eigenschaft ausgewählt aus der Gruppe bestehend aus: Feuchtigkeit, insbesondere relative Luftfeuchtigkeit, Druck, insbesondere Luftdruck, und Temperatur, insbesondere Lufttemperatur.

Es wird explizit betont, dass der Sensor mehr als drei Sensorelemente aufweisen kann, beispielsweise vier, fünf oder mehr Sensorelemente.

Im Rahmen des kompletten Trainings des neuronalen Netzes erfolgt erfindungsgemäß eine Optimierung der Betriebsparameter der einzelnen Sensorelemente, so dass die Zeit bis zum Vorliegen des Messwertes und der Fehler zwischen gemessener und tatsächlicher H₂-Konzentration in Luft minimal werden. Hier wird erfindungsgemäß die Temperatur des MOX-Elements, das typischerweise für den Betrieb beheizt wird, oder die Temperatur des Messelements des Wärmeleitfähigkeits-Sensorelements als zu trainierender Parameter gewählt.

Das erste Sensorelement, das zweite Sensorelement und das dritte Sensorelement können voneinander getrennte Sensorelemente sein. Alternativ können das erste Sensorelement, das zweite Sensorelement und das dritte Sensorelement in einem Sensorchip integrierte Sensorelemente sein.

Die Sensorelemente müssen entsprechend nicht zwangsläufig als physisch getrennte Komponenten vorliegen, die durch einen Schaltungsträger verbunden sind. Es können auch Sensor-Funktionen in einer Komponente bzw. Chip integriert sein. So können beispielsweise das Sensorelement für Wärmeleitfähigkeit und das Element mit MOX in einem Chip oder einem Modul integriert sein. Ebenso können die Sensorelemente für Luftfeuchte, Druck und Temperatur in einem Modul oder einem Chip integriert sein. Bevorzugt sind alle denkbaren Aggregationen von einzelnen oder mehreren Funktionen in einem Modul.

Der Sensor kann weiterhin einen Spannungswandler umfassen. Der Spannungswandler kann zum Verbinden mit einer externen Spannungsquelle ausgebildet sein. Der Spannungswandler kann weiterhin zum Erzeugen einer Versorgungsspannung für das erste Sensorelement, das zweite Sensorelement und das dritte Sensorelement ausgebildet sein.

Der Spannungswandler erhält somit von außen die Versorgungsspannung für den Gesamtsensor und generiert hieraus die für die Sensorelemente benötigte Versorgungsspannungen.

Der Sensor kann weiterhin eine Schnittstelle umfassen, wobei die Schnittstelle zum Empfangen von Steuerungsbefehlen von einer externen Steuereinheit und/oder zum Ausgeben von Messdaten des Sensors an eine externe Steuereinheit ausgebildet ist. Die Schnittstelle ist beispielsweise ein Datenaustauschbaustein. Der Datenaustauschbaustein empfängt Steuerbefehle für den Gesamtsensor von außen und gibt Messdaten zur Wasserstoffkonzentration und sonstige angeforderte Messdaten oder Metadaten nach außen. Ein solcher Datenaustauschbaustein ist auch als Kommunikationschip bekannt.

Der Sensor kann weiterhin ein Sensorgehäuse umfassen. Das erste Sensorelement, das zweite Sensorelement, das dritte Sensorelement und die elektronische Auswerteeinheit können in dem Sensorgehäuse angeordnet sein. Das Sensorgehäuse kann mindestens eine Öffnung aufweisen, mittels derer das erste Sensorelement, das zweite Sensorelement, das dritte Sensorelement dem fluiden Medium aussetzbar sind.

Über eine gasdurchlässige Öffnung im Sensorgehäuse wird somit das Gas, dessen Wasserstoffkonzentration zu messen ist, in das Gehäuse gebracht und dort den Sensorelementen zugeführt.

Die elektronische Auswerteeinheit ist zum Ausgeben von Steuer- und Korrektur-Signalen an das erste Sensorelement, das zweite Sensorelement, das dritte Sensorelement ausgebildet sein, um die Betriebsparameter zu verändern.

Somit umfasst der Sensor mehrere Sensorelemente und eine zentrale elektronische Verarbeitungseinheit in einem Sensorgehäuse, wobei die zentrale elektronische Verarbeitungseinheit die Signale der Sensorelemente mit Methoden des Machine Learnings verarbeitet, um daraus einen Messwert für die vorliegende H₂-Konzentation innerhalb < 1s mit geringem Fehler <0.1%vol H₂ zu ermitteln, der über die Ausgabeschnittstelle des Sensors dem Nutzer bereit gestellt wird. Dabei ist ebenfalls vorgesehen, dass von der zentralen elektronischen Verarbeitungseinheit im Rahmen der Signalverarbeitung auch Steuer- bzw. Korrektur-Signale an die Sensorelemente geleitet werden, um dadurch die Sensor-Gesamtperformance zu verbessern bzw. die Genauigkeit bzw. Ansprechzeit zu optimieren.

Unter einem Sensor wird im Rahmen der vorliegenden Erfindung grundsätzlich eine beliebige Vorrichtung verstanden, welche die mindestens eine Eigenschaft des fluiden Mediums erfassen kann und welche beispielsweise mindestens ein Messsignal entsprechend der erfassten Eigenschaft erzeugen kann, beispielsweise ein elektrisches Messsignal wie beispielsweise eine Spannung oder einen Strom. Das Messsignal kann dabei von einem oder mehreren Sensorelementen als Bauteile des Sensors ausgegeben werden. Bei der Eigenschaft kann es sich beispielsweise um eine physikalische und/oder eine chemische Eigenschaft handeln. Auch Kombinationen von Eigenschaften können erfassbar sein. Insbesondere kann der Sensor ausgestaltet sein zur Erfassung mindestens einer Eigenschaft eines Gases, insbesondere eines H₂-Anteils in einem Messgas. Auch andere Eigenschaften und/oder Kombinationen von Eigenschaften können erfassbar sein.

Der Sensor kann insbesondere zum Einsatz in einem Wasserstoff-Brennstoffzellenfahrzeug eingerichtet sein. Bei dem Messraum kann es sich grundsätzlich um einen beliebigen, offenen oder geschlossenen, Raum handeln, in welchem das fluide Medium, insbesondere das Messgas, aufgenommen ist, und/oder welcher von dem fluiden Medium, insbesondere dem Messgas, durchströmt wird.

Unter einem Gehäuse wird im Rahmen der vorliegenden Erfindung grundsätzlich ein beliebiges Bauteil oder eine Gruppe von Bauteilen verstanden, welche das Sensorelement ganz oder teilweise umschließen und/oder nach außen abschließen und dem Sensorelement eine mechanische Stabilität verleihen können. Insbesondere kann ein Gehäuse mindestens einen Innenraum umschließen. Beispielsweise kann das Gehäuse den Innenraum zumindest teilweise umschließen und ihn gegen seine Umgebung zumindest teilweise abgrenzen. Das Gehäuse kann insbesondere ganz oder teilweise aus mindestens einem der folgenden Materialien hergestellt sein: einem einem Kunststoff; einem Metall, einer Keramik oder einem Glas.

### Kurze Beschreibung der Zeichnungen

Weitere optionale Einzelheiten und Merkmale der Erfindung ergeben sich aus der nachfolgenden Beschreibung bevorzugter Ausführungsbeispiele, welche in den Figuren schematisch dargestellt sind.

Es zeigen:
Figur 1 eine schematische Darstellung eines erfindungsgemäßen Sensors zur Erfassung mindestens einer Eigenschaft eines fluiden Mediums in mindestens einem Messraum und
Figur 2 eine schematische Darstellung der elektronischen Auswerteeinheit.

### Ausführungsformen der Erfindung

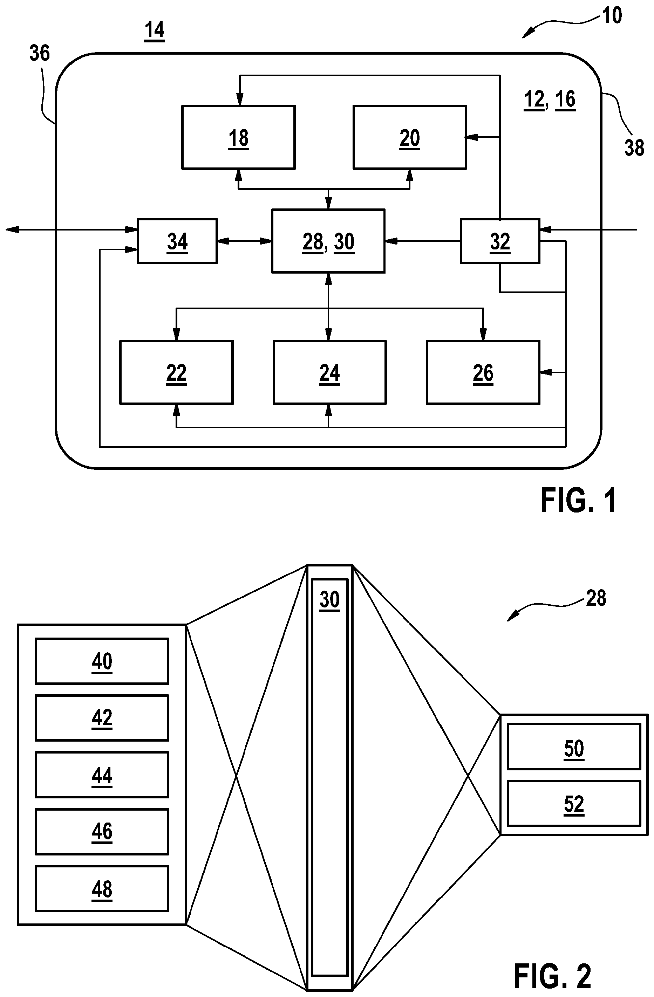

Figur 1 zeigt eine schematische Darstellung eines erfindungsgemäßen Sensors 10 zur Erfassung mindestens einer Eigenschaft eines fluiden Mediums 12 in mindestens einem Messraum 14, insbesondere zur Erfassung eines H₂-Anteils in einem Messgas 16. Der Sensor 10 kann insbesondere zum Einsatz in einem Wasserstoff-Brennstoffzellenfahrzeug eingerichtet sein. Auch andere Anwendungen sind jedoch möglich. Der Sensor 10 kann insbesondere ein oder mehrere in den Figuren nicht dargestellte, weitere Funktionselemente umfassen, wie beispielsweise Elektroden, Elektrodenzuleitungen und Kontakte, mehrere Schichten oder andere Elemente. Entsprechend kann der Sensor 10 im Abgasstrang des Wasserstoff-Brennstoffzellenfahrzeugs angebracht werden (exhaust) oder unter atmosphärischen Bedingungen arbeiten (ambient). Folglich kann es sich bei dem Messraum um einen Abgasstrang oder Innenraum des Wasserstoff-Brennstoffzellenfahrzeugs handeln.

Der Sensor 10 weist ein erstes Sensorelement 18 auf. Das erste Sensorelement 18 ist zum Erfassen einer Wärmeleitfähigkeit des fluiden Mediums 12 ausgebildet. Das erste Sensorelement 18 ist weiterhin zum Ausgeben eines ersten Messsignals ausgebildet.

Der Sensor 10 weist weiterhin ein zweites Sensorelement 20 auf. Das zweite Sensorelement 20 ist ein MOX-Sensorelement. Somit weist das zweite Sensorelement 20 ein halbleitendes Metalloxid auf. Das zweite Sensorelement 20 ist weiterhin zum Ausgeben eines zweiten Messsignals ausgebildet.

Der Sensor 10 weist weiterhin ein drittes Sensorelement 22 auf. Das dritte Sensorelement 22 ist zum Erfassen einer physikalischen Eigenschaft des fluiden Mediums ausgebildet. Das dritte Sensorelement 22 unterscheidet sich hinsichtlich der erfassten physikalischen Eigenschaft von dem ersten Sensorelement 18 und dem zweiten Sensorelement 20. Bei der gezeigten Ausführungsform ist das dritte Sensorelement 22 zum Erfassen von Feuchtigkeit des fluiden Mediums 12 ausgebildet. Beispielsweise ist das dritte Sensorelement 22 zum Erfassen einer relativen Luftfeuchtigkeit ausgebildet. Das dritte Sensorelement 22 ist zum Ausgeben eines dritten Messsignals ausgebildet.

Der Sensor 10 weist bei der gezeigten Ausführungsform mehr als drei Sensorelemente auf. So weist der Sensor 10 weiterhin ein viertes Sensorelement 24 auf. Das vierte Sensorelement 24 ist zum Erfassen einer physikalischen Eigenschaft des fluiden Mediums ausgebildet. Das vierte Sensorelement 24 unterscheidet sich hinsichtlich der erfassten physikalischen Eigenschaft von dem ersten Sensorelement 18 und dem zweiten Sensorelement 20. Bei der gezeigten Ausführungsform ist das vierte Sensorelement 24 zum Erfassen einer Temperatur des fluiden Mediums 12 ausgebildet. Beispielsweise ist das vierte Sensorelement 24 zum Erfassen einer Lufttemperatur ausgebildet. Das vierte Sensorelement 24 ist zum Ausgeben eines vierten Messsignals ausgebildet.

Der Sensor 10 weist weiterhin ein fünftes Sensorelement 26 auf. Das fünfte Sensorelement 26 ist zum Erfassen einer physikalischen Eigenschaft des fluiden Mediums ausgebildet. Das fünfte Sensorelement 26 unterscheidet sich hinsichtlich der erfassten physikalischen Eigenschaft von dem ersten Sensorelement 18 und dem zweiten Sensorelement 20. Bei der gezeigten Ausführungsform ist das fünfte Sensorelement 26 zum Erfassen eines Drucks des fluiden Mediums 12 ausgebildet. Beispielsweise ist das fünfte Sensorelement 26 zum Erfassen eines Luftdrucks ausgebildet. Das fünfte Sensorelement 26 ist zum Ausgeben eines fünften Messsignals ausgebildet.

Die Sensorelemente 18, 20, 22, 24, 26 können voneinander getrennte Sensorelemente sein. Bevorzugt sind die Sensorelemente 18, 20, 22, 24, 26 in einem nicht näher gezeigten Sensorchip oder Sensormodul integrierte Sensorelemente.

Der Sensor 10 weist weiterhin eine elektronische Auswerteeinheit 28 auf. Die elektronische Auswerteeinheit 28 ist zum Auswerten des ersten Messsignals, des zweiten Messsignals und des dritten Messsignals ausgebildet. Weiterhin ist die elektronische Auswerteeinheit 28 zum Auswerten des vierten Messsignals und des fünften Messsignals ausgebildet. Zu diesem Zweck kommuniziert die elektronische Auswerteeinheit 28 mit den Sensorelementen 18, 20, 22, 24, 26. Die elektronische Auswerteeinheit 28 ist weiterhin zum Verändern von Betriebsparametern des ersten Sensorelements 18, des zweiten Sensorelements 20 und/oder des dritten Sensorelements 24 ausgebildet ist. Weiterhin ist die elektronische Auswerteeinheit 28 zum Verändern von Betriebsparametern des vierten Sensorelements 24 und des fünften Sensorelements 26 ausgebildet. Die elektronische Auswerteeinheit 28 ist zum Verändern von Betriebsparametern des ersten Sensorelements 18, des zweiten Sensorelements 20, des dritten Sensorelements 22, des vierten Sensorelements 24 und des fünften Sensorelements 26 mittels eines Algorithmus 30 ausgebildet. Der Algorithmus 30 umfasst ein künstlich neuronales Netzwerk. Dabei wird für das künstlich neuronale Netzwerk auf Methoden des Machine Learning zurückgegriffen. Die elektronische Auswerteeinheit 28 ist zum Ausgeben von Steuer- und/oder Korrektur-Signalen an die Sensorelemente 18, 20, 22, 24, 26 ausgebildet, um deren Betriebsparameter zu verändern bzw. anzupassen.

Der Sensor 10 weist weiterhin einen Spannungswandler 32 auf. Der Spannungswandler 32 ist zum Verbinden mit einer externen Spannungsquelle (nicht näher gezeigt) ausgebildet. Der Spannungswandler 32 ist weiterhin zum Erzeugen einer Versorgungsspannung für die Sensorelemente 18, 20, 22, 24, 26 ausgebildet. Mit anderen Worten, liefert der Spannungswandler 32 an die Sensorelemente 18, 20, 22, 24, 26 die jeweils benötigte Versorgungsspannung.

Der Sensor 10 weist weiterhin eine Schnittstelle 34 auf. Die Schnittstelle 34 ist zum Empfangen von Steuerungsbefehlen von einer externen Steuereinheit (nicht näher gezeigt) ausgebildet. Die Schnittstelle 34 ist weiterhin zum Ausgeben von Messdaten des Sensors 10 an die externe Steuereinheit ausgebildet. Entsprechend kann die Schnittstelle 34 als Datenaustauschbaustein ausgebildet sein.

Der Sensor 10 weist weiterhin ein Sensorgehäuse 36 auf. Die Sensorelemente 18, 20, 22, 24, 26 und die elektronische Auswerteeinheit 28 sind in dem Sensorgehäuse 36 angeordnet. Weiterhin sind der Spannungswandler 32 und die Schnittstelle 34 zumindest teilweise in dem Sensorgehäuse 36 angeordnet. Das Sensorgehäuse 36 weist mindestens eine Öffnung 38 auf. Mittels der Öffnung 38 sind die Sensorelemente 18, 20, 22, 24, 26 dem fluiden Medium aussetzbar und von diesem kontaktierbar.

Figur 2 zeigt eine schematische Darstellung der elektronischen Auswerteeinheit 28. Wie bereits erwähnt, läuft in der elektronischen Auswerteeinheit ein Algorithmus 30, der ein künstlich neuronales Netzwerk umfasst. Figur 2 zeigt insbesondere eine schematische Darstellung des Algorithmus 30. Dem Algorithmus 30 werden erste Messdaten 40 von dem ersten Sensorelement 18, zweite Messdaten 42 von dem zweiten Sensorelement 20, dritte Messdaten 44 von dem dritten Sensorelement 22, vierte Messdaten 46 von dem vierten Sensorelement 24 und fünfte Messdaten 48 von dem fünften Sensorelement 46 zugeführt. Mittels des Algorithmus 30 wird aus den Messdaten 40, 42, 44, 46, 48 die H₂-Konzentration 50 in beispielsweise Vol.-% und die prozentuale Erreichung der Explosionsgrenze 52 in Prozent von H₂ in Luft ermittelt.

Nachstehend wird die Betriebsweise des Sensors 10 beschrieben. Neben den Sensorelementen 18, 20, 22, 24, 26 und der elektronischen Auswerteeinheit 28 als zentrale elektronische Signalverarbeitungseinheit ist der Spannungswandler 32 und die Schnittstelle 34 als Datenaustauschbaustein im Sensor 10 enthalten. Der Spannungswandler 32 erhält von außen die Versorgungsspannung für den gesamten Sensor 10 und generiert hieraus die für die Sensorelemente 18, 20, 22, 24, 26 benötigte Versorgungsspannung. Der Datenaustauschbaustein 34 empfängt Steuerbefehle für den gesamten Sensor 10 von außen und gibt Messdaten zur Wasserstoffkonzentration und sonstige angeforderte Messdaten oder Metadaten nach außen. Über die gasdurchlässige Öffnung 38 im Sensorgehäuse 36 wird das Messgas 16, dessen Wasserstoffkonzentration zu messen ist, in das Sensorgehäuse 36 gebracht und dort den Sensorelementen 18, 20, 22, 24, 26 zugeführt.

Die Information über die zu messende H₂-Konzentration im zugeführten Messgas 16 wird hauptsächlich durch das erste Sensorelement 18 als Wärmeleitfähigkeit-Sensorelement und das zweite Sensorelement 20 als MOX-Sensorelement generiert. Die Wärmeleitfähigkeit eines Gases ist invers proportional zur Wurzel der Masse der Gasmoleküle, so dass Gase mit leichten Atomen wie z.B. H₂-Moleküle oder He-Atome deutlich höhere Wärmeleitfähigkeit haben als Luft, dass im Wesentlichen aus N₂ und O₂-Molekülen besteht. Je höher die gemessene Wärmeleitfähigkeit, desto größer der Anteil an leichten Molekülen. Das zweite Sensorelement 20 weist auf oder besteht aus einem halbleitenden Metall-Oxid wie z.B. SnO₂ oder WO₃, dessen elektrischer Widerstand sich verringert, wenn ein chemisch reduzierend wirkendes Gas wie z.B. Wasserstoff, Methan oder Wasserdampf in Luft enthalten ist. Über den gemessenen elektrischen Widerstand, lässt sich dadurch der Gehalt an reduzierenden Gasen in Luft bestimmen. Wie beschrieben sind die Signale des ersten Sensorelements 18 und zweiten Sensorelement 20 nicht eindeutig durch die Wasserstoffkonzentration in Luft bestimmt, denn auch andere Gase in Luft können zum gleichen Messergebnis führen. Man spricht hier von Querempfindlichkeit bezüglich anderer Gase, welche die absolute Sensor-Genauigkeit begrenzt. Um eine angestrebte Genauigkeit von 0.1%vol. für H₂ bei allen nur denkbaren Luftzusammensetzungen gewährleisten zu können, sind Korrekturen in der Signalverarbeitung notwendig, indem zusätzliche Messgrößen erfasst und ausgewertet werden. Erfindungsgemäß werden Sensorelemente 22, 24, 26 für die relative Luftfeuchtigkeit, die Gastemperatur und den Gasdruck im H₂-Sensor integriert, deren Signale in der elektronischen Auswerteeinheit 28 berücksichtigt werden. Diese Berücksichtigung erfolgt allerdings nicht in Form eines Kennlinienfeldes, sondern durch Methoden des Machine Learnings wie z.B. durch Trainieren eines Neuronalen Netzes des Algorithmus 30, wie es schematisch in Fig. 2 dargestellt ist. Das Training des neuronalen Netzes erfolgt vor Auslieferung des Sensors 10. Dabei wird von den Methoden des Transfer-Learnings gebraucht gemacht, in der Art, dass das komplette zeitaufwändige Training des neuronalen Netzes nur an wenigen Produktsensoren erfolgt. Die übrigen Sensor-Produkte erhalten diese Knoten-Parameter bei ihrer Programmierung, und durchlaufen nur ein kurzes Training zum Zweck eines individuellen Fine-Tunings. Das Training besteht darin, den Sensor 10 nacheinander Luft mit verschiedenen Anteilen an Gasen wie vor allem H₂ oder Störgasen wie z.B. He oder CH₄ bei verschiedenen relativen Feuchtigkeiten und unterschiedlichen Luftdrücken und Temperaturen auszusetzen, und die eingestellten Parameter für die Gaskonzentrationen, relative Luftfeuchtigkeit, Luftdruck und Temperatur als Trainingsdaten für das neuronale Netz zu verwenden.

Im Rahmen des kompletten Trainings des neuronalen Netzes erfolgt erfindungsgemäß eine Optimierung der Betriebsparameter der einzelnen Sensorelemente 18, 20, 22, 24, 26, so dass die Zeit bis zum Vorliegen des Messwertes und der Fehler zwischen gemessener und tatsächlicher H₂-Konzentration in Luft minimal werden. Hier wird erfindungsgemäß die Temperatur des zweiten Sensorelements 20, das typischerweise für den Betrieb beheizt wird, oder die Temperatur des Messelements des ersten Sensorelements 18 als zu trainierender Parameter gewählt.

Der erfindungsgemäße Sensor 10 ist durch das Vorhandensein eines Sensorelements für Wärmeleitfähigkeit und eines Sensorelements mit halbleitendem Metalloxid sowie mindestens eines Sensorelements für eine andere physikalische Größe wie z.B. für relative Feuchte, Druck und Temperatur sowie einer zentralen elektronischen Signalverarbeitungseinheit nachweisbar. Weiterhin ist die Erfindung im Produkt in Betrieb daran erkennbar, dass sich die Betriebsparameter für die Sensorelemente an die äußeren Bedingungen anpassen und dadurch immer die Anforderungen an die Messgenauigkeit für H₂-%vol. und die Ansprechzeit erfüllen. In der zentralen elektronischen Signalverarbeitungseinheit sind Schaltungselemente bzw. -einheiten erkennbar, die für neuronale Netze optimiert sind.

## Patentansprüche

1. Sensor (10) zur Erfassung mindestens einer Eigenschaft eines fluiden Mediums (12) in mindestens einem Messraum (14), insbesondere zur Erfassung eines H₂-Anteils in einem Messgas (16), mindestens umfassend:
ein erstes Sensorelement (18), das zum Erfassen einer Wärmeleitfähigkeit des fluiden Mediums und zum Ausgeben eines ersten Messsignals ausgebildet ist,
ein zweites Sensorelement (20), das ein halbleitendes Metalloxid aufweist und das zum Ausgeben eines zweiten Messsignals ausgebildet ist,
ein drittes Sensorelement (22) zum Erfassen einer physikalischen Eigenschaft des fluiden Mediums, wobei sich das dritte Sensorelement (22) hinsichtlich der erfassten physikalischen Eigenschaft von dem ersten Sensorelement (18) und dem zweiten Sensorelement (20) unterscheidet und zum Ausgeben eines dritten Messsignals ausgebildet ist, und
eine elektronische Auswerteeinheit (28) zum Auswerten des ersten Messsignals, des zweiten Messsignals und dritten Messsignals, wobei die elektronische Auswerteeinheit (28) weiterhin zum Ausgeben von Steuersignalen an das erste Sensorelement (18), das zweite Sensorelement (20) und das dritte Sensorelement (22) und zum Verändern von Betriebsparametern des ersten Sensorelements, des zweiten Sensorelements (20) und/oder des dritten Sensorelements (22) mittels eines Algorithmus ausgebildet ist, **dadurch gekennzeichnet, dass** der Algorithmus (30) ein künstlich neuronales Netzwerk umfasst, wobei die elektronische Auswerteeinheit (28) zum Ausgeben von Korrektursignalen an das erste Sensorelement (18), das zweite Sensorelement (20), das dritte Sensorelement (22) zum Verändern der Betriebsparameter ausgebildet ist.

2. Sensor (10) nach einem der vorhergehenden Ansprüche, wobei das dritte Sensorelement (22) ausgebildet ist zur Erfassung mindestens einer physikalischen Eigenschaft ausgewählt aus der Gruppe bestehend aus: Feuchtigkeit, insbesondere relative Luftfeuchtigkeit, Druck, insbesondere Luftdruck, und Temperatur, insbesondere Lufttemperatur.

3. Sensor (10) nach einem der vorhergehenden Ansprüche, wobei das erste Sensorelement (18), das zweite Sensorelement (20) oder das dritte Sensorelement (22) voneinander getrennte Sensorelemente sind.

4. Sensor (10) nach einem der Ansprüche 1 bis 3, wobei das erste Sensorelement (18), das zweite Sensorelement (20) oder das dritte Sensorelement (22) in einem Sensorchip integrierte Sensorelemente sind.

5. Sensor (10) nach einem der vorhergehenden Ansprüche, weiterhin umfassend einen Spannungswandler (32), wobei der Spannungswandler (32) zum Verbinden mit einer externen Spannungsquelle ausgebildet ist, wobei der Spannungswandler (32) weiterhin zum Erzeugen einer Versorgungsspannung für das erste Sensorelement (18), das zweite Sensorelement (20) und das dritte Sensorelement (22) ausgebildet ist.

6. Sensor (10) nach einem der vorhergehenden Ansprüche, weiterhin umfassend eine Schnittstelle (34), wobei die Schnittstelle (34) zum Empfangen von Steuerungsbefehlen von einer externen Steuereinheit und/oder zum Ausgeben von Messdaten des Sensors an eine externe Steuereinheit ausgebildet ist.

7. Sensor (10) nach einem der vorhergehenden Ansprüche, weiterhin umfassend ein Sensorgehäuse (36), wobei das erste Sensorelement (18), das zweite Sensorelement (20), das dritte Sensorelement (22) und die elektronische Auswerteeinheit (28) in dem Sensorgehäuse (36) angeordnet sind, wobei das Sensorgehäuse (36) mindestens eine Öffnung (38) aufweist, mittels derer das erste Sensorelement (18), das zweite Sensorelement (20), das dritte Sensorelement (22) dem fluiden Medium aussetzbar sind.

## Claims

1. Sensor (10) for detecting at least one property of a fluid medium (12) in at least one measurement space (14), in particular for detecting an H₂ fraction in a measurement gas (16), comprising at least: a first sensor element (18), which is designed to detect a thermal conductivity of the fluid medium and to output a first measurement signal,
a second sensor element (20), which comprises a semiconducting metal oxide and is designed to output a second measurement signal,
a third sensor element (22) for detecting a physical property of the fluid medium, the third sensor element (22) differing from the first sensor element (18) and the second sensor element (20) in terms of the detected physical property and being designed to output a third measurement signal, and
an electronic evaluation unit (28) for evaluating the first measurement signal, the second measurement signal and the third measurement signal, the electronic evaluation unit (28) furthermore being designed to output control signals to the first sensor element (18), the second sensor element (20) and the third sensor element (22) and to change operating parameters of the first sensor element, the second sensor element (20) and/or the third sensor element (22) by means of an algorithm, **characterized in that** the algorithm (30) comprises an artificial neural network, the electronic evaluation unit (28) being designed to output correction signals to the first sensor element (18), the second sensor element (20) and the third sensor element (22) to change the operating parameters.

2. Sensor (10) according to one of the preceding claims, the third sensor element (22) being designed to detect at least one physical property selected from the group consisting of: moisture, in particular relative humidity, pressure, in particular air pressure, and temperature, in particular air temperature.

3. Sensor (10) according to either of the preceding claims, the first sensor element (18), the second sensor element (20) or the third sensor element (22) being separate sensor elements.

4. Sensor (10) according to one of Claims 1 to 3, the first sensor element (18), the second sensor element (20) or the third sensor element (22) being sensor elements integrated in a sensor chip.

5. Sensor (10) according to one of the preceding claims, furthermore comprising a voltage converter (32), the voltage converter (32) being designed for connection to an external voltage source, the voltage converter (32) furthermore being designed to generate a supply voltage for the first sensor element (18), the second sensor element (20) and the third sensor element (22).

6. Sensor (10) according to one of the preceding claims, furthermore comprising an interface (34), the interface (34) being designed to receive control commands from an external control unit and/or to output measurement data of the sensor to an external control unit.

7. Sensor (10) according to one of the preceding claims, furthermore comprising a sensor housing (36), the first sensor element (18), the second sensor element (20), the third sensor element (22) and the electronic evaluation unit (28) being arranged in the sensor housing (36), the sensor housing (36) having at least one opening (38) by means of which the first sensor element (18), the second sensor element (20) and the third sensor element (22) can be exposed to the fluid medium.

## Revendications

1. Capteur (10) pour détecter au moins une propriété d'un milieu fluide (12) dans au moins une chambre de mesure (14), en particulier pour détecter une fraction de H₂ dans un gaz de mesure (16), comprenant au moins : un premier élément capteur (18) qui est conçu pour détecter une conductivité thermique du milieu fluide et pour délivrer en sortie un premier signal de mesure, un deuxième élément capteur (20) qui comporte un oxyde métallique semiconducteur et qui est conçu pour délivrer en sortie un deuxième signal de mesure,
un troisième élément capteur (22) pour détecter une propriété physique du milieu fluide, le troisième élément capteur (22) différant du premier élément capteur (18) et du deuxième élément capteur (20) en ce qui concerne la propriété physique détectée et étant conçu pour délivrer en sortie un troisième signal de mesure, et
une unité d'évaluation électronique (28) pour évaluer le premier signal de mesure, le deuxième signal de mesure et le troisième signal de mesure, l'unité d'évaluation électronique (28) étant en outre conçue pour délivrer en sortie des signaux de commande au premier élément capteur (18), au deuxième élément capteur (20) et au troisième élément capteur (22) et pour modifier des paramètres de fonctionnement du premier élément capteur, du deuxième élément capteur (20) et/ou du troisième élément capteur (22) au moyen d'un algorithme, **caractérisé en ce que** l'algorithme (30) comprend un réseau neuronal artificiel, l'unité d'évaluation électronique (28) étant conçue pour délivrer en sortie des signaux de correction au premier élément capteur (18), au deuxième élément capteur (20), au troisième élément capteur (22) afin de modifier les paramètres de fonctionnement.

2. Capteur (10) selon l'une des revendications précédentes, dans lequel le troisième élément capteur (22) est conçu pour détecter au moins une propriété physique choisie dans le groupe constitué par : l'humidité, en particulier l'humidité relative de l'air, la pression, en particulier la pression atmosphérique, et la température, en particulier la température de l'air.

3. Capteur (10) selon l'une des revendications précédentes, dans lequel le premier élément capteur (18), le deuxième élément capteur (20) ou le troisième élément capteur (22) sont des éléments capteurs séparés les uns des autres.

4. Capteur (10) selon l'une des revendications 1 à 3, dans lequel le premier élément capteur (18), le deuxième élément capteur (20) ou le troisième élément capteur (22) sont des éléments capteurs intégrés dans une puce de capteur.

5. Capteur (10) selon l'une des revendications précédentes, comprenant en outre un convertisseur de tension (32), le convertisseur de tension (32) étant conçu pour être connecté à une source de tension externe, le convertisseur de tension (32) étant en outre conçu pour générer une tension d'alimentation pour le premier élément capteur (18), le deuxième élément capteur (20) et le troisième élément capteur (22).

6. Capteur (10) selon l'une des revendications précédentes, comprenant en outre une interface (34), l'interface (34) étant conçue pour recevoir des ordres de commande d'une unité de commande externe et/ou pour délivrer en sortie des données de mesure du capteur à une unité de commande externe.

7. Capteur (10) selon l'une des revendications précédentes, comprenant en outre un boîtier de capteur (36), le premier élément capteur (18), le deuxième élément capteur (20), le troisième élément capteur (22) et l'unité d'évaluation électronique (28) étant disposés dans le boîtier de capteur (36), le boîtier de capteur (36) présentant au moins une ouverture (38) au moyen de laquelle le premier élément capteur (18), le deuxième élément capteur (20) et le troisième élément capteur (22) peuvent être exposés au milieu fluide.
